(19) Europäisches Patentamt
European Patent Office
Office européen des brevets

(11) **EP 4 032 236 B1**

(12) **EUROPÄISCHE PATENTSCHRIFT**

(45) Veröffentlichungstag und Bekanntmachung des Hinweises auf die Patenterteilung:
**21.02.2024 Patentblatt 2024/08**

(21) Anmeldenummer: **20761188.0**

(22) Anmeldetag: **20.08.2020**

(51) Internationale Patentklassifikation (IPC):
***H04L 12/40*** *(2006.01)*

(52) Gemeinsame Patentklassifikation (CPC):
**H04L 12/40013**

(86) Internationale Anmeldenummer:
**PCT/EP2020/073367**

(87) Internationale Veröffentlichungsnummer:
**WO 2021/052705 (25.03.2021 Gazette 2021/12)**

(54) **ULTRASCHALLSYSTEM UND BILDGEBENDES ULTRASCHALLVERFAHREN**

ULTRASONIC SYSTEM AND IMAGING ULTRASONIC METHOD

SYSTÈME ÉCHOGRAPHIQUE ET PROCÉDÉ D'IMAGERIE ÉCHOGRAPHIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.09.2019 DE 102019124811**

(43) Veröffentlichungstag der Anmeldung:
**27.07.2022 Patentblatt 2022/30**

(73) Patentinhaber: **H-Next GmbH**
**91052 Erlangen (DE)**

(72) Erfinder: **HEID, Oliver**
**91052 Erlangen (DE)**

(74) Vertreter: **Meyer, Rudolf**
**Meyer & Döring**
**Partnerschaft mbB Patentanwälte**
**Nürnberger Straße 49**
**91052 Erlangen (DE)**

(56) Entgegenhaltungen:
**WO-A1-2010/127917      DE-A1-102011 006 827**
**DE-A1-102018 203 444      US-A1- 2012 177 059**

**Beschreibung**

Die Erfindung betrifft ein insbesondere als medizintechnisches System verwendbares Ultraschallsystem sowie ein bildgebendes Ultraschallverfahren.

Ein System und ein Verfahren zur Bildgebung mittels Ultraschall ist beispielsweise aus der US 2013 / 0079639 A1 bekannt. Das bekannte Ultraschallsystem umfasst eine Mehrzahl an elektroakustischen Wandlern, welche Ultraschallsignale senden sowie Echos der gesendeten Ultraschallsignale empfangen. Die elektroakustischen Wandler sind in einem Schallkopf angeordnet. Erfasste analoge Signale werden digitalisiert, wobei digitale Rohdaten weiterverarbeitet werden. Zur Weiterleitung von Daten wird ein Bussystem vorgeschlagen. Digitalisierte Daten können gespeichert und unter anderem durch einen Empfangs-Strahlformer gespeichert werden, um letztlich darstellbare Ultraschallbilder zu generieren.

Ein weiteres Ultraschallsystem mit einer Ultraschallsonde, das heißt einem Schallkopf, und einer hiervon räumlich getrennten Datenverarbeitungseinheit ist in der EP 2 614 775 A1 beschrieben. Zwischen der Ultraschallsonde und der Datenverarbeitungseinheit werden Daten über eine drahtlose Schnittstelle übermittelt.

Aus der US 2008/0 110 266 A1 ist ein Ultraschallsystem mit einer über eine kabellose oder kabelgebundene Schnittstelle an einen Rechner gekoppelten Ultraschallsonde bekannt. Die Ultraschallsonde weist Vorverstärker, A/D-Wandler sowie einen Pufferspeicher auf. Zur Fokussierung ausgestrahlter akustischer Signale werden akustische und synthetische Verfahren vorgeschlagen. Zudem ist eine synthetische Fokussierung empfangener Ultraschallsignale vorgesehen.

Im Fall eines aus der US 2008 / 0 294 046 A1 beschriebenen Ultraschallsystems ist ein Schallkopf über ein Kabel mit einer batteriebetriebenen Auswerteeinheit verbunden.

Die US 2008 / 0 110 266 A1 beschreibt ein Ultraschallsystem mit einem Array aus Schallwandlern, die als piezoelektrische Wandler ausgebildet und über Vorverstärker an Analog-Digital-Wandler angeschlossen sind. Die von den Analog-Digital-Wandlern gelieferten digitalen Signale werden teilweise bereits innerhalb des Schallkopfes weiterverarbeitet. Eine weitere digitale Signalverarbeitung erfolgt in einer Haupteinheit, die über Kabel oder drahtlos mit dem Schallkopf verbunden ist.

Eine Speicherarchitektur eines bildgebenden Ultraschallsystems ist im Detail in der US 2014 / 0 058 266 A1 beschrieben. Zu verarbeitende Daten werden in diesem Fall über ein Datennetzwerk übertragen.

Die EP 2 627 257 B1 beschreibt ein Ultraschallbildgebungssystem mit einer konkav gekrümmten Ultraschallwandleranordnung. In der Ultraschallwandleranordnung befinden sich mehrere Sendeöffnungen, welche einen Streuer mit unkonzentrierten Ultraschall-Pings beschallen. Mit den Ultraschallwandlern ist ein Steuersystem verknüpft, welches unter anderem das Umschalten zwischen den verschiedenen Sendeöffnungen ermöglicht.

Die EP 3 117 774 B1 offenbart ein Softwarebasiertes Ultraschallbildgebungssystem, innerhalb dessen von einem Schema mit direktem Speicherzugriff (DMA = direct memory access) Gebrauch gemacht wird. Hierbei ist eine Frontend-Einheit des Ultraschallbildgebungssystems zum Übertragen von Kanaldaten in einem DMA-Schema vorgesehen.

Aus der US 2008/0 151 765 A1 ist ein Ringpuffer zur Vermeidung des Jitters bei einer Übertragung von Audiodaten bekannt. Der Ringpuffer ist zur Durchführung voneinander unabhängiger Lese- und Schreibvorgänge eingerichtet.

Die DE 10 2018 203444 A1 ein Bussystem zur Kopplung von Sensoren mit Verarbeitungs- und Speichereinheiten eines Rechners. Die Sensoren sind einfache Ultraschallsensoren, die direkt über einen analog zu digital Wandler ihre Daten kontinuierlich, also als Stream, durch ein echtzeitfähiges Bussystem übertragen. Die Busübertragung erfolgt zentral über einen Buscontroller. Dieser Buscontroller leitet die Daten an die Speicher- und Verarbeitungseinheiten. Die Verarbeitungseinheiten führen einfache Berechnungen durch und übertragen die Daten danach über das Bussystem zu den Speichereinheiten, die als Festplatte ausgelegt sind.

Der Erfindung liegt die Aufgabe zugrunde, die Bildgebung mittels Ultraschall gegenüber dem genannten Stand der Technik insbesondere unter dem Gesichtspunkt einer effizienten und sicheren Datenverarbeitung weiterzuentwickeln.

Diese Aufgabe wird erfindungsgemäß gelöst durch ein Ultraschallsystem mit den Merkmalen des Anspruchs 1. Ebenso wird die Aufgabe gelöst durch ein bildgebendes Ultraschallverfahren gemäß Anspruch 10. Im Folgenden im Zusammenhang mit dem Ultraschallverfahren erläuterte Ausgestaltungen und Vorteile der Erfindung gelten sinngemäß auch für die Vorrichtung, das heißt das Ultraschallsystem, und umgekehrt.

Das Ultraschallsystem arbeitet mit einer beweglichen, insbesondere als Handheld-Gerät ausgebildeten Ultraschallsonde, welche eine Mehrzahl an Sende- und Empfangselementen umfasst, sowie mit einer Datenverarbeitungseinheit, welche mit der Ultraschallsonde leitungsgebunden oder nicht leitungsgebunden gekoppelt ist. In jedem Fall ist die Datenverarbeitungseinheit von der Ultraschallsonde physikalisch getrennt, das heißt ohne festgelegte geometrische Relation von der Ultraschallsonde beabstandet. In typischen Ausgestaltungen ist zwischen der Ultraschallsonde und der Datenverarbeitungseinheit eine Datenverbindung per Kabel hergestellt. Die Ultraschallsonde umfasst in jedem Fall eine Anzahl Sende-Endstufen, Sende-Empfangsweichen, analoger Vorverstärker und Analog-Digital-Wandler, sowie mindestens eine digitale Anschlusskomponente, über die die Ultraschallsonde datentechnisch mit der Datenverarbeitungseinheit gekoppelt ist. Eine Datenverarbeitung, insbesondere eine Strahlformung (Beamforming), ist dagegen in der Ultraschallsonde nicht vorgesehen.

Die Datenverarbeitungseinheit des Ultraschallsystems weist zwei als Ringspeicher konzipierte Speicher, nämlich einen Empfangsspeicher und einen Sendespeicher, welche zusammenfassend auch als Ringpuffer bezeichnet werden, sowie eine zur Generierung von Ultraschallbildern ausgebildete Recheneinheit auf. Hierbei stellen die Ringspeicher Komponenten eines die Ultraschallsonde sowie die genannte Datenverbindung, insbesondere ein Kabel, umfassenden Echtzeit-Bussystems (RTS) dar. Zugleich sind die Ringspeicher in ein außerhalb des Echtzeit-Bussystems (RTS) existierendes, zum Betrieb der Recheneinheit vorgesehenes Betriebssystem (BS), insbesondere Standard-Betriebssystem, eingebunden.

Ein System, welches Daten innerhalb der Ultraschallsonde leitet und mit Hilfe eines in die Ultraschallsonde eingebauten Taktgebers (clock) getaktet ist, wird als Sonden-System (Son-S) bezeichnet. Die Taktungenauigkeit (Jitter), das heißt Ausführungszeitungenauigkeit, des Sonden-Systems (Son-S) ist um mindestens drei Größenordnungen geringer als die Taktungenauigkeit (Jitter) des Echtzeit-Bussystems (RTS). Das heißt, dass das Sonden-System (Son-S) mit einer weit höheren zeitlichen Präzision als das Echtzeit-Bussystem (RTS) arbeitet. Gleichzeitig ist die Reaktionszeit des Betriebssystems (BS) der Recheneinheit um mindestens zwei Größenordnungen länger als die Taktungenauigkeit (Jitter) des Echtzeit-Bussystems. Somit unterscheidet sich die Reaktionszeit des Betriebssystems (BS) von der Taktungenauigkeit der Ultraschallsonde um mindestens den Faktor $10^5$. Trotz dieser fünf Größenordnungen, die jeweils eine Zeitungenauigkeit oder Zeitdifferenz betreffen, werden die vom Ultraschallsystem generierten Bilder als Echtzeitbilder wahrgenommen. Über das Echtzeit-Bussystem (RTS) werden Sende- und Empfangsdaten zwischen der Ultraschallsonde und der von dieser beabstandeten Datenverarbeitungseinheit gleichzeitig per Streaming ununterbrochen mit im Durchschnitt konstanter Rate in getakteter Weise bei zustandslosem, nicht zwischen Sende- und Empfangsbetrieb unterscheidendem Betrieb der Ultraschallsonde übertragen.

Insgesamt weist das erfindungsgemäße Ultraschallverfahren, innerhalb dessen ein zu untersuchendes Objekt Ultraschallpulsen ausgesetzt wird und Echosignale aufgenommen werden, folgende Merkmale auf:

- Bereitstellung einer Ultraschallsonde, welche eine Mehrzahl an Sende- und Empfangselementen umfasst, sowie einer mit dieser datentechnisch gekoppelten Datenverarbeitungseinheit, welche einen Hauptprozessor sowie einen weiteren Prozessor, insbesondere in Form eines Graphikprozessors, mit einer Mehrzahl an Rechenwerken umfasst,
- Generierung von zum Betrieb der Sende- und Empfangselemente vorgesehenen Ansteuerungsdaten durch die Datenverarbeitungseinheit, wobei die Ansteuerungsdaten unter einem keine harten Echtzeitanforderungen erfüllenden Standardbetriebssystem erzeugt und asynchron - bezogen auf ihre Erzeugung - in einen als Ringspeicher konzipierten Sendespeicher, der einem Echtzeit-Bussystem zuzurechnen ist, geschrieben werden,
- Asynchrones - bezogen auf die Generierung und das Speichern der Ansteuerungsdaten - Übermitteln von Ansteuerungsdaten aus dem Sendespeicher an die Ultraschallsonde, wobei die Ultraschallsonde als Komponente des Echtzeit-Bussystems betrieben wird und die Ansteuerdaten mit einer im Durchschnitt konstanten ersten Datenübertragungsrate ohne Pause per Streaming übermittelt werden, und wobei die Taktungenauigkeit (Jitter) eines Komponenten innerhalb der Ultraschallsonde umfassenden Sonden-Systems um mindestens drei Größenordnungen geringer und die Reaktionszeit des Standardbetriebssystems um mindestens zwei Größenordnungen länger als die Taktungenauigkeit (Jitter) des Echtzeit-Bussystems ist,
- Ultraschallpulse werden nach Maßgabe der Ansteuerdaten mit Hilfe von in der Ultraschallsonde angeordneten Sende-Endstufen erzeugt, wobei die von den Sende-Endstufen betätigten Sende- und Empfangselemente insbesondere als Piezo-Elemente ausgebildet sind,
- Die zugehörigen Echosignale werden innerhalb der Ultraschallsonde analog verstärkt und synchron, ohne Unterbrechung und mit konstanter Taktfrequenz digitalisiert und per Streaming in Echtzeit lückenlos ohne weitere Verarbeitung über eine Datenverbindung, insbesondere ein Kabel, asynchron, bezogen auf die Digitalisierung, in einen von der Ultraschallsonde räumlich getrennten, als Ringspeicher konzipierten, dem Echtzeit-Bussystem zuzurechnenden Empfangsspeicher geschrieben, wobei die Datenübermittlung zum Empfangsspeicher ununterbrochen mit einer im Wesentlichen konstanten zweiten Datenübertragungsrate erfolgt, welche von der genannten ersten Datenübertragungsrate, die sich auf die Datenübermittlung von der Datenverarbeitungseinheit zur Ultraschallsonde bezieht, um nicht mehr als einen Faktor fünf, insbesondere um nicht mehr als den Faktor zwei, abweicht,
- Eine Auswahl von im Empfangsspeicher eingegangenen Daten wird, vom Hauptprozessor gesteuert, als Messdatenblöcke auf mehrere Rechenwerke verteilt, wobei die einzelnen Messdatenblöcke jeweils einer Gruppe an Empfangselementen zugeordnet sind,
- Mehrere Messdatensätze werden parallel und asynchron zum Ringspeicher-Schreibvorgang des Echtzeit-Bussystems durch Rechenwerke verarbeitet, die jeweils durch den unter dem Standardbetriebssystem laufenden Hauptprozessor gesteuert werden, wobei die Verarbeitung jeweils die Empfangs-Strahlformung und die Bildrekonstruktion umfasst und mit Hilfe eines jeden Rechenwerks ein Teilbild rekonstruiert wird,

- Aus den Teilbildern wird durch die Auswerteeinheit ein Gesamtbild als auf einem Bildschirm anzuzeigendes Ultraschallbild generiert.

Innerhalb der Ultraschallsonde ist ein einziger Taktgeber (Clock) für die Sende-Endstufen sowie die Analog-Digital-Wandler vorgesehen.

Die Erfindung geht von der Überlegung aus, dass beim Betrieb eines Ultraschallsystems, welches insbesondere in der Medizintechnik einsetzbar sein soll, tendenziell steigende Anforderungen an die Qualität der Ultraschallbilder, welche typischerweise Schnittbilder darstellen, zu erfüllen sind. Die Qualität bezieht sich hierbei sowohl auf die Auflösung der Bilder als auch auf die Abmessungen des mit einer einzigen Ultraschallaufnahme erfassbaren Bereichs, wobei die Abmessungen die beispielsweise in cm angegebene Tiefe des unterhalb der Ultraschallsonde liegenden, simultan erfassten Untersuchungsbereichs einschließen.

Eine höhere Qualität von Ultraschallaufnahmen geht typischerweise mit einer höheren Rate an zu übertragenden und zu verarbeitenden Daten einher. Es sind verschiedenste physikalische Prinzipien der Signalübertragung denkbar, unter anderem eine optische Signalübertragung, wie beispielsweise in der EP 0 762 142 B1 beschrieben. Um die Rate der zwischen einer Ultraschallsonde und einer gesonderten Datenverarbeitungsanlage zu übertragenden Daten zu begrenzen, ist eine Vorverarbeitung und Selektion von Daten innerhalb der Ultraschallsonde ein denkbarer Weg. Jede Verarbeitung von Daten in der Ultraschallsonde ist jedoch zwangsläufig mit Energieverbrauch und damit Wärmeentwicklung verbunden. Zudem spielt der Raumbedarf von Datenverarbeitungskomponenten eine Rolle. Ein Ausweg aus diesem Dilemma könnte in einer teilweisen analogen Datenverarbeitung innerhalb der Ultraschallsonde gesucht werden, was auch unter dem Gesichtspunkt hoher Echtzeitanforderungen von Vorteil sein könnte.

Mit der Erfindung wird indes ein anderer Weg eingeschlagen, indem die Ultraschallsonde, sowohl was das Empfangen als auch was das Senden von Daten betrifft, zustandslos betrieben wird. Sende- und Empfangselemente werden hierbei mit kurzer Taktung von beispielsweise 8 ns fortlaufend auf einen jeweils aktuellen Zustand eingestellt. Eine Beibehaltung dieses Zustandes, solange keine neue Einstellung erfolgt, ist nicht vorgesehen. Vielmehr wird mit jedem Takt, im genannten Beispiel alle 8 ns, erneut ein Zustand eingestellt, wobei dieser mit dem vorherigen Zustand identisch sein oder einen abweichenden Zustand darstellen kann. Damit wird einerseits der Datenverarbeitungsaufwand, was die Komplexität der Verarbeitung betrifft, in der Ultraschallsonde minimiert und zum anderen das Risiko der Beibehaltung einer Fehleinstellung im laufenden Betrieb eliminiert. Beim Einschalten des Ultraschallsystems sowie beim Abreißen der Datenverbindung zwischen der Ultraschallsonde und der Datenverarbeitungseinheit nehmen die Sende- und Empfangselemente einen sicheren Zustand ein, sofern dieser nicht ohnehin bereits gegeben ist. Die sehr hohe Datenrate, die mit dem zustandslosen Betrieb der Sende- und Empfangselemente verbunden ist, wird in Kauf genommen.

Bereits bei der analogen Verarbeitung der empfangenen Daten, das heißt Ultraschallechos, kann bei dem Ultraschallsystem der Aufwand gegenüber herkömmlichen Lösungen dadurch minimiert werden, dass die Vorverstärker mit konstanter Verstärkung (gain) betrieben werden. Dies bedeutet, dass keine Abhängigkeit der Vorverstärkung von der Distanz einer ein Echo auslösenden Struktur vom Sende- und Empfangselement gegeben ist. Somit wird bei einem Bildpunkt, welcher die geringste Distanz zum Sende- und Empfangselement aufweist, dieselbe Verstärkung angewandt, die auch bei Bildpunkten mit der maximalen Entfernung vom Sende- und Empfangselement zur Anwendung kommt.

Der weitgehende Entfall von Datenverarbeitung innerhalb der Ultraschallsonde ermöglicht trotz sehr hoher kontinuierlicher Datenraten, sowohl was die Datenübermittlung zur Ultraschallsonde als auch was die Datenübermittlung von der Ultraschallsonde zur Auswerteeinheit betrifft, eine Gestaltung der Ultraschallsonde als Hand-Held-Gerät mit geringer, ohne besondere Kühlungsmaßnahmen beherrschbarer Wärmeentwicklung.

Bei den Sende- und Empfangselementen der Ultraschallsonde handelt es sich in bevorzugter Ausgestaltung um kombinierte Sende- und Empfangselemente, das heißt Elemente, die sowohl die Sende- als auch die Empfangsfunktion übernehmen. Insbesondere sind hierfür Piezo-Elemente geeignet.

Was die Anzahl und geometrische Anordnung der Sende- und Empfangselemente, insbesondere Piezo-Elemente, innerhalb der Ultraschallsonde betrifft, sind verschiedenste Ausgestaltungen realisierbar. Beispielsweise sind in grundsätzlich bekannter Gestaltung 128 Piezo-Elemente linienförmig angeordnet.

Jedem Sende- und Empfangselement ist in bevorzugter Ausgestaltung eine Sende-Empfangsweiche vorgeschaltet, welche zur getakteten Beaufschlagung des Sende- und Empfangselements für jeweils genau einen Takt mit einem von n Codes ausgebildet ist, wobei n mindestens vier ist und (n-1) Sendecodes und zusätzlich genau ein Empfangscode vorgesehen sind. Im Fall von drei Sendecodes entspricht beispielsweise einer dieser Sendecodes einem positiven Spannungswert, ein weiterer Sendecode einem betragsmäßig gleichen negativen Spannungswert und der dritte Sendecode der Spannung Null. Die Codierung kann in diesem Fall durch ein 2 Bit Signal erfolgen. Trotz des sehr kurzen Taktes von beispielsweise 8 ns bleibt damit die Datenübertragungsrate in einem mit vertretbarem Aufwand beherrschbaren Bereich. Mit einer höheren Anzahl an Bits ist theoretisch auch die Einstellung von Zwischenwerten der Spannung, welche an ein Sende- und Empfangselement anzulegen ist, möglich. In jedem Fall wird jeder Sende-Empfangs-Zustand aktiv und nur einen Takt lang aufrechterhalten. Im Fehlerfall erfolgt eine automatische Default-Sicherheits-Abschaltung, das heißt die Einnahme eines sicheren Default-Zustands.

Innerhalb der Datenverarbeitungseinheit befindet sich in vorteilhafter Ausgestaltung ein Root Complex, über welchen das Echtzeit-Bussystem eine Verbindung zur Ultraschallsonde herstellt. Unter anderem dient der Root Complex der Übertragung von Message-Datenpaketen (MSI), die Sende- sowie Empfangsdaten betreffen, wobei durch die Message-Datenpakete (MSI) zählende Semaphore zum zeitlichen Blockieren und Freigeben der streamenden Datenübertragung in den Sendespeicher sowie aus dem Empfangsspeicher realisiert sind. Die zur Übertragung von Message-Datenpaketen (MSI) bereitgehaltene Datenübertragungskapazität beträgt vorzugsweise nicht mehr als jeweils 1% derjenigen Datenübertragungskapazitäten, welche zur Übertragung einerseits von Ansteuerdaten und andererseits von Messdaten über die Datenverbindung bereitgestellt sind.

Durch das Betriebssystem (BS) der Recheneinheit, das Echtzeit-Bussystem (RTS) und das Sonden-System (Son-S) sind drei Zeitdomänen gegeben, welche - in der genannten Reihenfolge der Systeme - steigende Echtzeitanforderungen erfüllen. Vorzugsweise ist zwischen dem zeitlichen Jitter (TSon) des Sonden-Systems (Son-S), dem Jitter (TBus) des Echtzeit-Bussystems (Bus-S), und der Reaktionszeit (TBS) des Betriebssystems (BS) folgender Zusammenhang gegeben:

$$10^1 \leq \frac{TBus^2}{TSoN * TBS} \leq 10^4$$

Insbesondere hat der genannte, dimensionslose Bruch $TBus^2$ / (TSon x TBS) einen Wert von mindestens 100 und höchstens 10.000.

Der Empfangsdatenspeicher, in welchen Daten über das Echtzeit-Bussystems (Bus-S) geschrieben werden, und aus welchem Daten zur Verarbeitung unter dem Betriebssystem (BS), insbesondere Standard-Betriebssystem, gelesen werden, ist vorzugsweise dazu ausgebildet, Empfangsdatenblöcke, welche Sendedatensätzen zugeordnet sind, für mindestens 20 ms zu speichern. Hierbei ist der Sendedatenstrom zur andauernden, ununterbrochenen Ansteuerung der Sende- und Empfangselemente vorgesehen, wobei die zugehörigen, in den Sende- und Empfangsspeichern abgelegten Empfangsdaten zeitlich lückenlos, sequentiell und ohne in jedem Einzelfall zugehöriges Zeitsignal gespeichert sind. Zeitinformationen werden auf diese Weise implizit, durch die Größe der gespeicherten Datenblöcke sowie die Reihenfolge der Speicherung, das heißt die Speicheradresse, innerhalb des Ultraschallsystems weitergegeben.

Die Datenverbindung, welche die Ultraschallsonde mit der Datenverarbeitungseinheit koppelt, ist beispielsweise Teil einer PCIe-Verbindung. Insbesondere ist die Datenverbindung durch ein Kabel realisiert. Die PCIe-Verbindung ermöglicht einen Speicherdirektzugriff (DMA) auf den Empfangsspeicher und den Sendespeicher. Über ein und dieselbe PCIe-Verbindung können Ansteuerdaten in der einen Richtung und zeitgleich Messdaten in der anderen Richtung sowie zusätzliche Datenpakete, insbesondere die bereits genannten Message-Datenpakete (MSI), übertragen werden, wobei das Datenvolumen der Message-Datenpakete (MSI) in bevorzugter Ausgestaltung um mindestens zwei Größenordnungen kleiner als das Volumen der über die Ringspeicher geleiteten Daten ist.

Beim Betrieb des Ultraschallsystems werden in bevorzugter Verfahrensführung durch ein Hauptprogramm zwei nebenläufige Prozesse gestartet. Einer dieser Prozesse füllt den Sendespeicher; der andere Prozess vergibt Rekonstruktions-Kommandos an den zusätzlichen Prozessor, insbesondere Graphik-Prozessor. Zwei verschiedene Message-Datenpakete (MSI) sind derart an ein Dateisystem angehängt, dass zählende Semaphore realisiert werden. Diese Semaphore werden durch die beiden Prozesse periodisch gelesen, womit zum einen das Schreiben in den Sendespeicher blockiert wird, solange die Ultraschallsonde nicht genügend Daten aus dem Sendespeicher entnimmt, und zum anderen die Entnahme von Daten aus dem Empfangsspeicher blockiert wird, solange die Ultraschallsonde nicht genügend Daten in den Empfangsspeicher geschrieben hat. Mit diesen bedarfsweisen Blockaden wird eine Synchronisation in der Größenordnung eines 1 ms Niveaus umgesetzt, was ausreichend schnell für eine als flüssig wahrgenommene Bildschirmanzeige ist. Von dem 1 ms Niveau, welches mittels des Standard-Betriebssystems erreichbar ist, ist die harte Echtzeitanforderungen erfüllende, lediglich rudimentäre Datenverarbeitung innerhalb der Ultraschallsonde auf einem größenordnungsmäßigen 10 ps Niveau zu unterscheiden. Das zwischen diesen beiden Extremen liegende Echtzeit-Bussystem (RTS) ermöglicht die bidirektionale Übertragung ausreichend großer Datenströme zwischen der Ultraschallsonde und der Datenverarbeitungseinheit, wobei die von der Ultraschallsonde gelieferten Messdaten, abgesehen von der Digitalisierung, unverarbeitet übertragen werden.

Die Ultraschallsonde ist als aktive Sonde im Unterschied zum Stand der Technik dazu in der Lage, erstens zum Betrieb der Sende- und Empfangselemente benötigte Daten selbsttätig aus dem der Datenverarbeitungseinrichtung zuzurechnenden Sendespeicher anzufordern und im Rahmen des Echtzeit-Bussystems (RTS) zu nutzen und zweitens Messdaten selbständig in den ebenfalls der Datenverarbeitungseinrichtung zuzurechnenden Empfangsspeicher zu schreiben.

Nachfolgend wird ein Ausführungsbeispiel der Erfindung anhand einer Zeichnung näher erläutert. Hierin zeigen:

Fig. 1   ein Ultraschallsystem in einer schematischen Übersichtsdarstellung,

Fig. 2    in einem Schaubild ein mit dem Ultraschallsystem nach Fig. 1 ausführbares Verfahren.

Ein insgesamt mit 1 bezeichnetes Ultraschallsystem umfasst eine Ultraschallsonde 2 und eine hiermit datentechnisch verbundene Datenverarbeitungseinheit 3. Die Ultraschallsonde 2 wird beim Betrieb des Ultraschallsystems 1 in an sich bekannter Weise durch den Nutzer, das heißt in der Regel den Arzt, per Hand geführt. Die Datenübertragung zwischen der Ultraschallsonde 2 und der Datenverarbeitungseinheit 3, die sich beispielsweise in einem fahrbaren Gestell befindet, erfolgt im Ausführungsbeispiel über eine Datenleitung 14, das heißt ein Kabel, unter Nutzung des PCIe-Standards. Allgemein stellt das Kabel 14 eine Datenverbindung dar, welche abweichend vom Ausführungsbeispiel auch als optische Signalübertragung oder als Funkverbindung ausgeführt sein könnte.

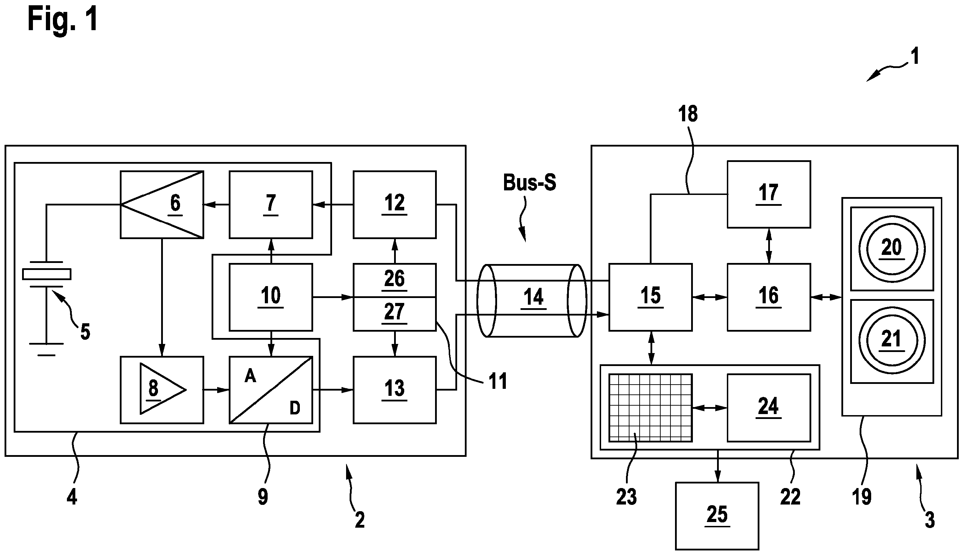

Innerhalb der Ultraschallsonde 2, welche als aktiver, zustandsfreie Ultraschall-Probenkopf gestaltet ist, befindet sich eine Anzahl an analogen Frontend-Einheiten 4, wobei die Anzahl der Frontend-Einheiten 4 durch die Anzahl der Sende-Empfangskanäle der Ultraschallsonde 2 vorgegeben ist. Ein Sende- und Empfangselement 5, das heißt Schallwandlerelement, innerhalb der Frontend-Einheit 4 ist in an sich bekannter Weise als Piezo-Element aufgebaut. Das Schallwandlerelement 5 ist an eine Sende-Empfangsweiche 6 angeschlossen. Die Sende-Empfangsweiche 6 wiederum ist einerseits mit einem Leistungssender 7, welcher auch als Sende-Endstufe bezeichnet wird, und andererseits mit einem Vorverstärker 8 verknüpft. Die Sende-Endstufe 7 ist im Ausführungsbeispiel zusammen mit der Empfangsweiche 6 durch ein einziges Bauteil realisiert. Vom Vorverstärker 8 gelieferte analoge Signale werden durch einen Analog-Digital-Wandler 9 in digitale Signale, das heißt Empfangssignale, umgesetzt. Vergleichbar mit dem kombinierten Bauteil 7, 8 ist im Ausführungsbeispiel auch die Funktion des Vorverstärkers 8 und des Analog-Digital-Wandlers 9 in einem einzigen Bauteil 8, 9 zusammengefasst. Generell dient die Aufteilung der Funktionselemente einerseits der Ultraschallsonde 2 und andererseits der Datenverarbeitungseinheit 3, wie in Fig. 1 skizziert, der Visualisierung. Tatsächlich sind innerhalb der Ultraschallsonde 2 sowie innerhalb der Datenverarbeitungseinheit 3 jeweils Kombinationen von Funktionselementen möglich.

Zur Taktung der Datenverarbeitung ist innerhalb der Ultraschallsonde 2 ein Taktgeber (Clock) 10, kurz auch als Uhr bezeichnet, vorgesehen. Die Uhr 10 befindet sich außerhalb des analogen Frontends 4, wobei sie datentechnisch sowohl mit den Leistungssendern 7 als auch mit den Analog-Digital-Wandlern 9 verknüpft ist. Darüber hinaus existieren datentechnische Verknüpfungen zwischen den Leistungssendern 7 und einem Sende-Serializer 12, sowie zwischen den Analog-Digital-Wandlern 9 und einem Empfangsdaten-Paketizer 13. Der Sende-Serializer 12 setzt Sendedaten in mit konstanter Rate getaktete, gestreamte kanalweise Bitströme zur Ansteuerung der Sende-Empfangsweichen 6 und der Schallwandlerelemente 5 um. Umgekehrt bewerkstelligt der Empfangsdaten-Paketizer 13 ein Streaming der von den Analog-Digital-Wandlern 9 erhaltenen ADC-Daten zu Empfangs-Datenpaketen.

Der Sende-Serializer 12 ist ebenso wie der Empfangsdaten-Paketizer 13 angeschlossen an eine Zähler-Anordnung 11, die zwei Ringpuffer-Adresszähler 26, 27 umfasst, nämlich einen Sendezweig 26 und einen Empfangszweig 27. Der Sendezweig 26 initiiert Datenpaket-Anforderungen (TLPs = Transaction layer packet) an einen Root Complex 15 zum Lesen aus einem Sendedatenspeicher 20, das heißt Sendespeicher, auf den noch näher eingegangen werden wird. In prinzipiell vergleichbarer Weise initiiert der Empfangszweig 27 die Übermittlung von Datenpaketen an einen Empfangsdatenspeicher 21, welcher kurz als Empfangsspeicher bezeichnet wird. Die Zähler-Anordnung 11, der Sende-Serializer 12 und der Empfangsdaten-Paketizer 13 werden zusammenfassend als digitale Anschlusskomponente der Ultraschallsonde 2 bezeichnet. Der Sendedatenspeicher 20 ist ebenso wie der Empfangsdatenspeicher 21 als Ringpuffer aufgebaut und einem Hauptspeicher 19 der Datenverarbeitungseinheit 3 zuzurechnen. Auch der Root Complex 15 befindet sich in der Datenverarbeitungseinheit 3.

Die Verbindung zwischen der Ultraschallsonde 2 und der Datenverarbeitungseinheit 3 ist durch ein Bussystem Bus-S hergestellt, welches als PCIe System realisiert ist und das Kabel 14 umfasst. Durch das Kabel 14, das heißt PCIe-Kabel, ist eine bidirektionale digitale Datenverbindung hergestellt. Über diese Datenverbindung werden beim Betrieb des Ultraschallsystems 1 Daten gleichzeitig mit hohen Raten in beide Richtungen übertragen, wie im Folgenden noch näher erläutert wird.

Der an das Kabel 14 angeschlossene Root Complex 15 ist innerhalb der Datenverarbeitungseinheit 3 an einen Hauptprozessor (CPU) 17, an eine Memory Management Unit (MNU) 16, sowie an einen Grafikprozessor 22 angeschlossen. Vom Root Complex 15 aus werden Signaldaten 18 direkt, unter Umgehung der Memory Management Unit 16, an den Hauptprozessor 17 übertragen. Die Signaldaten 18 sind im vorliegenden Fall Message Signaled Interrupts (MSI) als vom Root Complex 15 extrahierte Message-Datenpakete. Die MSI 18 dienen der Notifizierung von Adresszählerständen des Sendezweigs 26 sowie des Empfangszweigs 27. Bei Überschreiten festgelegter Grenzen, was die über den Sende-Serializer 12 sowie den Empfangsdaten-Paketizer 13 übertragenen aus dem Sendespeicher 20 entnommenen beziehungsweise in den Empfangsspeicher 21 geschriebenen Daten betrifft, werden die Werte von Zählern geändert. Mit diesen Zählern, die in bevorzugter Ausgestaltung größere Werte als 1 annehmen können und als zählende Semaphore fungieren, wird nicht nur das Befüllen und Entleeren der Ringpuffer 20, 21 gesteuert, sondern auch maßgeblich Einfluss auf die Zusammenwirkung zwischen den Hauptprozessor 17 und dem Grafikprozessor 22 genommen.

Der Grafikprozessor 22, welcher allgemein auch als Prozessor bezeichnet wird, umfasst eine Vielzahl, beispielsweise 4096, an Rechenwerken 23 sowie eine Speichereinheit 24. Mit Hilfe des Grafikprozessors 22 wird ein Anzeigegerät 25, nämlich ein zur Bildausgaberate synchronisierbarer Bildschirm, betrieben. Die Komponenten 15, 16, 17, 19, 22 der Datenverarbeitungseinheit 3 bilden zusammen eine Recheneinheit.

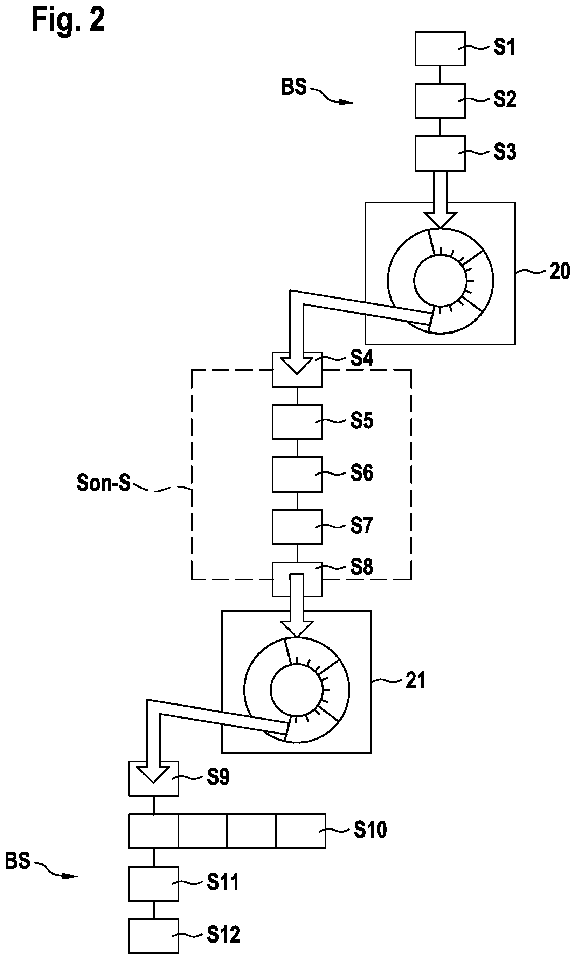

Das mit dem Ultraschallsystem 1 nach Figur 1 durchführbare Verfahren ist in Figur 2 veranschaulicht. Hierin sind insgesamt zwölf Verfahrensschritte mit S1 bis S12 bezeichnet. Als Schritt S1, das heißt Start des Verfahrens, ist die Bereitstellung der Ultraschallsonde 2 und der mit dieser über ein Echtzeit-Bussystem BUS-S verbundenen Datenverarbeitungseinheit 3 zu verstehen. Die Datenverarbeitungseinheit 3 wird mit einem an sich bekannten Standardbetriebssystem BS betrieben, das kurz als Betriebssystem bezeichnet wird. Das Betriebssystem BS hat typische Reaktionszeiten in der Größenordnung von 1 ms bis 10 ms und erfüllt damit keine harten Echtzeitanforderungen. Das Betriebssystem BS wird auch als 1 ms-Domäne bezeichnet. Im Betriebssystem BS werden im Schritt S2 Ansteuerdaten generiert, welche letztlich zur Ansteuerung der Schallwandlerelemente 5 benötigt werden. Die Ansteuerdaten werden im Schritt S3 in den Sendespeicher 20 geschrieben. Im Gegensatz zum Hauptprozessor 17 ist der Sendespeicher 20, ebenso wie der Empfangsspeicher 21, dem Echtzeit-Bussystem BUS-S zuzurechnen.

Im Schritt S4 werden Ansteuerdaten, ausgelöst durch die Ultraschallsonde 2, aus dem Sendespeicher 20 gelesen und über das Kabel 14, das heißt die Datenverbindung 14, an die Ultraschallsonde 2 übertragen. Das Echtzeit-Bussystem BUS-S, welches mit Hilfe des Kabels 14 sowie des Root Complex 15 aufgebaut ist, arbeitet mit einem Jitter in der Größenordnung von 10 ms und wird dementsprechend als 10 ms-Domäne bezeichnet.

Die aus dem Sendespeicher 20 gelesenen Daten werden per Streaming, praktisch ununterbrochen und mit konstanter Datenrate, an die zustandslose Ultraschallsonde 2 übermittelt. In der Ultraschallsonde 2 ist durch den Taktgeber 10 ein 8 ns-Takt vorgegeben, wobei die Taktungenauigkeit in der Größenordnung von 10 ps liegt. Die entsprechende Zeitdomäne im Sondensystem (Son-S) stellt dementsprechend eine 10 ps-Domäne dar.

Im Schritt S5 werden unter Nutzung der gestreamten Ansteuerdaten Ultraschallpulse durch die Sende- und Empfangselemente 5 erzeugt und Echos durch dieselben Elemente 5 empfangen. Die empfangenen Echos werden im Schritt S6 mit Hilfe der Vorverstärkter 8 mit konstanter Verstärkung (gain) verstärkt. Die Digitalisierung durch die Analog-Digital-Wandler 9 erfolgt im Schritt S7.

Die digitalisierten Daten werden im Schritt S8 per Streaming über das Kabel 14 übertragen und in den Empfangsspeicher 21 geschrieben. Das Übertragen von Daten über das Kabel 14 zur Datenverarbeitungseinheit 3 geschieht zeitgleich mit dem Empfangen von Daten durch die Ultraschallsonde 2 aus der Datenverarbeitungseinheit 3.

Asynchron zum Übertragungsvorgang werden im Schritt S9 Daten aus dem Empfangsspeicher 21 ausgelesen und im Schritt S10 auf die einzelnen Rechenwerke 23 verteilt. Das Generieren von Teilbildern im Schritt S11 durch die Rechenwerke 23 stellt eine Stahlformung (beam forming) dar. Die letztliche Bildgenerierung (image forming), das heißt das Zusammensetzen des Ultraschallbilds, erfolgt im Schritt S12.

Die Zeitspanne vom Empfangen der Ultraschallechos durch die Schallwandlerelemente 5 bis zum Ausgeben der Ultraschallbilder auf dem Anzeigegerät 25 beträgt weniger als 100 ms, so dass für den Benutzer der Eindruck des Betrachtens von Echtzeitbildern gegeben ist. Tatsächlich durchläuft die Datenverarbeitung in der beschriebenen Weise insgesamt drei Zeitdomänen, wobei in der Zeitdomäne mit der geringsten Taktungenauigkeit, das heißt im Sondensystem Son-S, die Datenverarbeitung auf ein Minimum reduziert, jedoch eine sehr hohe Datenrate von mehreren Gbit pro Sekunde gegeben ist.

Daten mit einer derart hohen Rate werden hierbei sowohl von der Ultraschallsonde 2 empfangen als auch von der Ultraschallsonde 2 gesendet. Das Volumen der pro Zeiteinheit empfangenen Daten unterscheidet sich vom Volumen der pro Zeiteinheit gesendeten Daten um nicht mehr als den Faktor fünf. Die sehr hohe Rate von der Ultraschallsonde 2 empfangener Daten hängt direkt mit dem zustandslosen Betrieb der Ultraschallsonde 2 zusammen. Das zur bidirektionalen Datenübertragung genutzte Kabel 14 weist zwar eine Taktungenauigkeit auf, welche die Präzision des Sondensystems Son-S um mehrere Größenordnungen verfehlt, jedoch im Vergleich zum Standardbetriebssystem BS vergleichsweise hohe Echtzeitanforderungen erfüllt.

Der gesamte Prozess der Strahlformung ist in die unter dem Betriebssystem BS laufende Datenverarbeitungseinheit 3 verlegt. Das bedeutet unter anderem, dass bei dem Auslösen eines einzigen sogenannten Schusses durch die Ultraschallsonde 2, das heißt beim Generieren eines Ultraschallsignals, dessen Echo auszuwerten ist, nicht lediglich, wie im Stand der Technik üblich, ein schmaler Teilbereich, das heißt eine oder wenige Bildspalten, betrachtet wird, sondern sämtliche Echodaten, die physikalisch einem Sendepuls zuzuordnen sind, aufgenommen und ohne digitale Verarbeitung übertragen, nämlich gestreamt, werden. Erst innerhalb der Datenverarbeitungseinheit 3 werden aus den aufgenommenen Daten Teilmengen extrahiert, die zur Generierung von Teilbildern und letztlich eines Gesamtbildes genutzt werden.

**Bezugzeichenliste**

| | |
|---|---|
| 1 | Ultraschallsystem |
| 2 | Ultraschallsonde |
| 3 | Datenverarbeitungseinheit |
| 4 | Analoges Frontend |
| 5 | Sende- und Empfangselement, Schallwandlerelement |
| 6 | Sende-Empfangsweiche |
| 7 | Leistungssender, Sende-Endstufe |
| 8 | Vorverstärker |
| 9 | Analog-Digital-Wandler |
| 10 | Taktgeber, Clock |
| 11 | Zähler-Anordnung |
| 12 | Sende-Serializer |
| 13 | Empfangsdaten-Paketizer |
| 14 | Datenverbindung, Kabel |
| 15 | Root Complex |
| 16 | Memory Management Unit |
| 17 | Hauptprozessor, CPU |
| 18 | MSI-Datenpaket, Signaldaten |
| 19 | Hauptspeicher |
| 20 | Sendespeicher, Ringspeicher |
| 21 | Empfangsspeicher, Ringspeicher |
| 22 | Prozessor, Graphikprozessor |
| 23 | Rechenwerk |
| 24 | Speicher |
| 25 | Anzeigegerät |
| 26 | Sendezweig der Zähler-Anordnung |
| 27 | Empfangszweig der Zähler-Anordnung |
| BS | Betriebssystem, Standardbetriebssystem |
| Bus-S | Echtzeit-Bussystem |
| Son-S | Sondensystem |
| S1 ... S12 | Verfahrensschritte |

**Patentansprüche**

1. Ultraschallsystem, mit einer Ultraschallsonde (2), welche eine Mehrzahl an Sende- und Empfangselementen (5) umfasst, sowie mit einer von der der Ultraschallsonde (2) physikalisch getrennten und mit dieser per Datenverbindung (14) verbundenen Datenverarbeitungseinheit (3), wobei die Ultraschallsonde (2) eine Anzahl analoger Vorverstärker (8), mehrere Analog-Digital-Wandler (9), sowie mindestens eine digitale Anschlusskomponente (11, 12, 13) aufweist, und die Datenverarbeitungseinheit (3) zwei Ringspeicher (20, 21), nämlich einen Empfangsspeicher (21) und einen Sendespeicher (20), sowie eine zur Generierung von Ultraschallbildern ausgebildete Recheneinheit (15, 16, 17, 19, 22) aufweist, und wobei zur datentechnischen Kopplung der Ultraschallsonde (2) mit der Recheneinheit (15, 16, 17, 19, 22) ein die genannte Datenverbindung (14) umfassendes Echtzeit-Bussystem (Bus-S) vorgesehen ist, in welches auch die Ringspeicher (20, 21) eingebunden sind und welches einerseits an ein Daten innerhalb der Ultraschallsonde (2) leitendes Sonden-System (Son-S) und andererseits an ein zum Betrieb der Recheneinheit (15, 16, 17, 19, 22) vorgesehenes Betriebssystem (BS) angebunden ist, und wobei die Taktungenauigkeit (Jitter) des Sonden-Systems (Son-S) um mindestens drei Größenordnungen geringer und die Reaktionszeit des Betriebssystems (BS) um mindestens zwei Größenordnungen länger als die Taktungenauigkeit (Jitter) des Echtzeit-Bussystems (Bus-S) ist, und wobei das Echtzeit-Bussystem (Bus-S) in Zusammenwirkung mit dem Sonden-System (Son-S) und dem Betriebssystem (BS) dazu ausgebildet ist, Sende- und Empfangsdaten gleichzeitig per Streaming ununterbrochen mit im Durchschnitt konstanter Rate getaktet bei zustandslosem, nicht zwischen Sende- und Empfangsbetrieb unterscheidendem Betrieb der Ultraschallsonde (2) zu übertragen.

2. Ultraschallsystem nach Anspruch 1, **dadurch gekennzeichnet, dass** durch die genannte Datenverbindung (14) eine Verbindung zwischen der Ultraschallsonde (2) und einem in die Datenverarbeitungseinheit (3) integrierten Root Complex (15) hergestellt ist, welcher unter anderem zur Übertragung von Message-Datenpaketen (MSI), die Sende- sowie Empfangsdaten betreffen, vorgesehen ist, wobei durch die Message-Datenpakete (MSI) zählende Semaphore zum zeitlichen Blockieren und Freigeben der streamenden Datenübertragung in den Sendespeicher (20) sowie aus dem Empfangsspeicher (21) realisiert sind.

3. Ultraschallsystem nach Anspruch 2, **dadurch gekennzeichnet, dass** jedem Sende- und Empfangselement (5) eine Sende-Empfangsweiche (6) vorgeschaltet ist, welche zur getakteten Beaufschlagung des Sende- und Empfangselements (5) für jeweils genau einen Takt mit einem von n Codes ausgebildet ist, wobei n mindestens vier ist und (n-1) Sendecodes, welchen Spannungswerte zugeordnet sind, und genau ein Empfangscode vorgesehen ist.

4. Ultraschallsystem nach Anspruch 2 oder 3, **dadurch gekennzeichnet, dass** die zur Übertragung von Message-Datenpaketen (MSI) bereitgehaltene Datenübertragungskapazität nicht mehr als jeweils 1% der Datenübertragungskapazitäten, welche zur Übertragung einerseits von Ansteuerdaten und andererseits von Messdaten über die Datenverbindung (14) bereitgestellt sind, beträgt.

5. Ultraschallsystem nach einem der Ansprüche 1 bis 4, **dadurch gekennzeichnet, dass** zwischen dem zeitlichen Jitter (TSon) des Sonden-Systems (Son-S), dem Jitter (TBus) des Echtzeit-Bussystems (Bus-S), und der Reaktionszeit (TBS) des Betriebssystems (BS) folgender Zusammenhang gegeben ist:

$$10^1 \leq \frac{TBus^2}{TSoN * TBS} \leq 10^4$$

6. Ultraschallsystem nach einem der Ansprüche 1 bis 5, **dadurch gekennzeichnet, dass** der Empfangsspeicher (21) dazu ausgebildet ist, Empfangsdatenblöcke, welche Sendedatensätzen zugeordnet sind, für mindestens 20 ms zu speichern.

7. Ultraschallsystem nach Anspruch 6, **dadurch gekennzeichnet, dass** der Sendedatenstrom zur andauernden, ununterbrochenen Ansteuerung der Sende- und Empfangselemente (5) vorgesehen ist und die zugehörigen, in den Sende- und Empfangsspeichern (20, 21) abgelegten Empfangsdaten zeitlich lückenlos, sequentiell und ohne in jedem Einzelfall zugehöriges Zeitsignal gespeichert sind.

8. Ultraschallsystem nach einem der Ansprüche 1 bis 7, **dadurch gekennzeichnet, dass** die Datenverbindung (14) zwischen der Ultraschallsonde (2) und den Ringspeichern (20, 21) als PCIe-Verbindung ausgebildet ist.

9. Ultraschallsystem nach Anspruch 8, **dadurch gekennzeichnet, dass** die Datenverbindung ein die Ultraschallsonde (2) mit der Datenverarbeitungseinheit (3) verbindendes Kabel (14) umfasst.

10. Bildgebendes Ultraschallverfahren, wobei ein zu untersuchendes Objekt Ultraschallpulsen ausgesetzt wird und Echosignale aufgenommen werden, mit folgenden Merkmalen:

   - Bereitstellung einer Ultraschallsonde (2), welche eine Mehrzahl an Sende- und Empfangselementen (5) umfasst, sowie einer mit dieser datentechnisch gekoppelten Datenverarbeitungseinheit (3), welche einen Hauptprozessor (17) sowie einen Prozessor (22), insbesondere Graphikprozessor, mit einer Mehrzahl an Rechenwerken (23) umfasst,
   - Generierung von zum Betrieb der Sende- und Empfangselemente (5) vorgesehenen Ansteuerungsdaten durch die Datenverarbeitungseinheit (3), wobei die Ansteuerungsdaten unter einem keine harten Echtzeitanforderungen erfüllenden Standardbetriebssystem (BS) erzeugt und asynchron - bezogen auf die Erzeugung - in einen als Ringspeicher konzipierten Sendespeicher (20), der einem Echtzeit-Bussystem (BUS-S) zuzurechnen ist, geschrieben werden,
   - Asynchrones - bezogen auf die Generierung und das Speichern der Ansteuerungsdaten - Übermitteln von Ansteuerungsdaten aus dem Sendespeicher (20) an die Ultraschallsonde (2), wobei die Ultraschallsonde (2) als Komponente des Echtzeit-Bussystems (BUS-S) betrieben wird und die Ansteuerdaten mit einer im Durchschnitt konstanten ersten Datenübertragungsrate ohne Pause per Streaming übermittelt werden, und wobei die Taktungenauigkeit (Jitter) eines Komponenten innerhalb der Ultraschallsonde umfassenden Sonden-Systems (Son-S) um mindestens drei Größenordnungen geringer und die Reaktionszeit des Standardbetriebssystems (BS) um mindestens zwei Größenordnungen länger als die Taktungenauigkeit (Jitter) des Echtzeit-Bussystems (Bus-S) ist,
   - Ultraschallpulse werden nach Maßgabe der Ansteuerdaten mit Hilfe von in der Ultraschallsonde (2) angeordneten Sende-Endstufen (7) erzeugt, wobei die von den Sende-Endstufen (7) betätigten Sende- und Empfangselemente (5) insbesondere als Piezo-Elemente ausgebildet sind,
   - Die zugehörigen Echosignale werden innerhalb der Ultraschallsonde (2) analog verstärkt und synchron, ohne Unterbrechung und mit konstanter Taktfrequenz digitalisiert und per Streaming in Echtzeit lückenlos ohne weitere Verarbeitung über eine Datenverbindung (14) asynchron in einen von der Ultraschallsonde (2) räumlich getrennten, als Ringspeicher konzipierten, dem Echtzeit-Bussystem (BUS-S) zuzurechnenden Empfangsspeicher (21) geschrieben, wobei die Datenübermittlung zum Empfangsspeicher (21) ununterbrochen mit einer im Wesentlichen konstanten zweiten Datenübertragungsrate erfolgt, welche von der ersten, die Datenübermittlung von der Datenverarbeitungseinheit (3) zur Ultraschallsonde (2) betreffenden Datenübertragungsrate um nicht mehr als einen Faktor fünf abweicht,
   - Eine Auswahl von im Empfangsspeicher (21) eingegangenen Daten wird, vom Hauptprozessor (17) gesteuert, als Messdatenblöcke auf mehrere Rechenwerke (23) verteilt, wobei die einzelnen Messdatenblöcke jeweils einer Gruppe an Empfangselementen (5) zugeordnet sind,
   - Mehrere Messdatensätze werden parallel und asynchron zum Schreibvorgang, das heißt zum Schreiben in den Empfangsspeicher (21), des Echtzeit-Bussystems (BUS-S) durch Rechenwerke (23) verarbeitet, die jeweils durch den unter dem Standardbetriebssystem (BS) laufenden Hauptprozessor (17) gesteuert werden, wobei die Verarbeitung jeweils die Empfangs-Strahlformung und die Bildrekonstruktion umfasst und mit Hilfe eines jeden Rechenwerks (23) ein Teilbild rekonstruiert wird,
   - Aus den Teilbildern wird durch die Datenverarbeitungseinheit (3) ein Gesamtbild als Ultraschallbild generiert.

11. Ultraschallverfahren nach Anspruch 10, **dadurch gekennzeichnet, dass** zusätzlich zu Ansteuerungsdaten und von der Ultraschallsonde (2) gelieferten Messdaten innerhalb der Datenverarbeitungseinheit (3) Message-Datenpakete (MSI) übertragen werden, welche zählende Semaphore zum Blockieren und Freigeben der streamenden, seriellen Datenübertragung in den Sendespeicher (20) sowie aus dem Empfangsspeicher (21) realisieren, wobei das Datenvolumen der Message-Datenpakete (MSI) um mindestens zwei Größenordnungen kleiner als das Volumen der über die Ringspeicher (20, 21) geleiteten Daten ist.

12. Ultraschallverfahren nach Anspruch 10 oder 11, **dadurch gekennzeichnet, dass** die Echosignale eines jeden Empfangskanals innerhalb der Ultraschallsonde (2) mit konstanter Verstärkung (gain) durch einen Vorverstärker (8) analog verstärkt werden.

13. Ultraschallverfahren nach einem der Ansprüche 10 bis 12, **dadurch gekennzeichnet, dass** in die Sende- und Empfangsspeicher (20, 21) Daten ohne explizite Zeitinformation geschrieben werden, wobei die Zeitinformation beim Auslesen der Daten implizit der Größe der gespeicherten Datenblöcke sowie der Reihenfolge der Speicherung, das heißt der Speicheradresse, entnommen wird.

14. Ultraschallverfahren nach einem der Ansprüche 10 bis 13, **dadurch gekennzeichnet, dass** die Ultraschallsonde (2) erstens zum Betrieb der Sende- und Empfangselemente (5) benötigte Daten selbsttätig aus dem Sendespeicher (20) anfordert und im Rahmen des Echtzeit-Bussystems (BUS-S) nutzt und zweitens Messdaten selbständig in den Empfangsspeicher (21) schreibt.

15. Ultraschallverfahren nach einem der Ansprüche 10 bis 14, **dadurch gekennzeichnet, dass** die Ultraschallsonde (2) beim Einschalten sowie beim Abreißen der Verbindung zur Datenverarbeitungseinheit (3) selbsttätig einen sicheren Default-Zustand einnimmt.

**Claims**

1. An ultrasound system, comprising an ultrasound probe (2), which comprises a plurality of transmission and reception elements (5), and also comprising a data processing unit (3) which is physically separated from the ultrasound probe (2) and is connected to the latter by means of data connection (14), wherein the ultrasound probe (2) comprises a number of analogue preamplifiers (8), a plurality of analog-digital converters (9), and at least one digital terminal component (11, 12, 13), and the data processing unit (3) comprises two ring buffers (20, 21), i.e. a reception buffer (21) and a transmission buffer (20), as well as a computing unit (15, 16, 17, 19, 22) constituted for generating ultrasound images, and wherein a real-time bus system (Bus-S) comprising the aforementioned data connection (14) is provided for the data coupling of the ultrasound probe (2) with the computing unit (15, 16, 17, 19, 22), in which the ring buffers (20, 21) are also incorporated, and which on the one hand is connected to a probe system (Son-S) transmitting data within the ultrasound probe (2) and on the other hand to an operating system (OS) provided for the operation of the computing unit (15, 16, 17, 19, 22), and wherein the clocking inaccuracy (jitter) of the probe system (Son-S) is at least three orders of magnitude less and the reaction time of the operating system (OS) is at least two orders of magnitude longer than the clocking inaccuracy (jitter) of the real-time bus system (Bus-S), and wherein the real-time bus system (Bus-S) is constituted in cooperation with the probe system (Son-S) and the operating system (OS), in order that transmission and reception data are transmitted simultaneously by streaming uninterrupted clocked on average with a constant rate with a stateless operation of the ultrasound probe (2) not differing between transmission and reception operation.

2. The ultrasound system according to claim 1, **characterised in that** a connection between the ultrasound probe (2) and a root complex (15) integrated into the data processing unit (3) is produced by the said data connection (14), which is provided amongst other things for the transmission of message data packets (MSI), which relate to transmission and reception data, wherein counting semaphores for the temporal blocking and releasing of the streaming data transmission into the transmission buffer (20) and out of the reception buffer (21) are implemented by the message data packets (MSI).

3. The ultrasound system according to claim 2, **characterised in that** a transmission-reception switch (6) is connected upstream of each transmission and each reception element (5), which switch is constituted for clocked application on the transmission and reception element (5) for in each case precisely one cycle with one of n codes, wherein n is at least four and (n-1) transmission codes, to which voltage values are assigned, and precisely one reception code is provided.

4. The ultrasound system according to claim 2 or 3, **characterised in that** the data transmission capacity kept ready for the transmission of message data packets (MSI) amounts in each case to not more than 1% of the data transmission capacities which are kept ready on the one hand for the transmission of control data and on the other hand of measurement data via the data connection (14).

5. The ultrasound system according to any one of claims 1 to 4, **characterised in that** the following relationship is present between the time jitter (TSon) of the probe system (Son-S), the jitter (TBus) of the real-time bus system (Bus-S), and the reaction time (TBS) of the operating system (OS):

$$10^1 \leq \frac{TBus^2}{TSoN * TBS} \leq 10^4$$

6. The ultrasound system according to any one of claims 1 to 5, **characterised in that** the reception buffer (21) is designed to store reception data blocks, which are assigned to transmission datasets, for at least 20 ms.

7. The ultrasound system according to claim 6, **characterised in that** the transmission data flow is provided for the ongoing, uninterrupted control of the transmission and reception elements (5) and the associated reception data stored in the transmission and reception buffers (20, 21) continuously, sequentially and without an associated time signal in each individual case.

8. The ultrasound system according to any one of claims 1 to 7, **characterised in that** the data connection (14) between the ultrasound probe (2) and the ring buffers (20, 21) is constituted as a PCIe connection.

9. The ultrasound system according to claim 8, **characterised in that** the data connection comprises a cable (14) connecting the ultrasound probe (2) to the data processing unit (3).

10. An imaging ultrasound method, wherein an object to be examined is subjected to ultrasound pulses and echo signals are picked up, with the following features:

- Provision of an ultrasound probe (2), which comprises a plurality of transmission and reception elements (5), as well as a data processing unit (3) coupled in terms of data with the latter, which comprises a main processor (17) and a processor (22), in particular a graphic processor, with a plurality of computing units (23),
- generation of control data by the data processing unit (3) which are provided for the operation of the transmission and reception elements (5), wherein the control data are generated under a standard operating system (OS) which does not meet any hard real-time requirements and are written asynchronously - relative to the generation - into a transmission buffer (20) designed as a ring buffer, which is to be ascribed to a real-time bus system (BUS-S),
- asynchronous - relative to the generation and the storage of the control data - transmission of control data from the transmission buffer (20) to the ultrasound probe (2), wherein the ultrasound probe (2) is operated as a component of the real-time bus system (BUS-S) and the control data are transmitted with a first data transmission rate constant on average without pause by streaming, and wherein the clocking inaccuracy (jitter) of a probe system (Son-S) which contains components inside the ultrasound probe is at least three orders of magnitude less and the reaction time of the standard operating system (OS) at least two orders of magnitude longer than the clocking inaccuracy (jitter) of the real-time bus system (BUS-S),
- ultrasound pulses are generated according to the control data with the aid of transmission endstages (7) arranged in the ultrasound probe (2), wherein the transmission and reception elements (5) actuated by the transmission endstages (7) are constituted in particular as piezoelements,
- the associated echo signals are amplified by analog means inside the ultrasound probe (2) and synchronously digitalised without interruption and with constant clock frequency and are written, by streaming in real-time gapless and without further processing, via a data connection (14), asynchronously into a reception buffer (21) separated spatially from the ultrasound probe (2), designed as a ring buffer and to be ascribed to the real-time bus system (BUS-S), wherein the data transmission to the reception buffer (21) takes place uninterrupted with an essentially constant second data transmission rate, which does not deviate by a factor of more than five from the first data transmission rate, which relates to the data transmission from the data processing unit (3) to the ultrasound probe (2),
- a selection from data received in the reception buffer (21) is distributed, controlled by the main processor (17), as measurement data blocks on a plurality of computing units (23), wherein the individual measurement data blocks are each assigned to a group of reception elements (5),
- a plurality of measurement datasets are processed in parallel and asynchronously to the writing process, i.e. to the writing into the reception buffer (21), of the real-time bus system (BUS-S) by computing units (23), which are each controlled by the main processor (17) running under the standard operating system (OS), wherein the processing in each case comprises the reception beamforming and the image reconstruction and a partial image is reconstructed with the aid of each computing unit (23),

- a total image is generated by the data processing unit (3) from the partial images as an ultrasound image.

**11.** The ultrasound method according to claim 10, **characterised in that,** in addition to control data and measurement data delivered by the ultrasound probe (2), message data packets (MSI) are transmitted inside the data processing unit (3), which implement counting semaphores for the blocking and releasing the streaming, serial data transmission into the transmission buffer (20) and out of the reception buffer (21), wherein the data volume of the message-data packets (MSI) is smaller by at least two orders of magnitude than the volume of the data transmitted via the ring buffers (20, 21).

**12.** The ultrasound method according to claim 10 or 11, **characterised in that** the echo signals of each reception channel are amplified inside the ultrasound probe (2) by analog means with a constant gain by means of a preamplifier (8).

**13.** The ultrasound method according to any one of claims 10 to 12, **characterised in that** data are written without explicit time information into transmission and reception buffers (20, 21), wherein the time information when the data are read out is extracted implicitly from the size of the stored data blocks and the sequence of the storage, i.e. the memory address.

**14.** The ultrasound method according to any one of claims 10 to 13, **characterised in that** the ultrasound probe (2) in the first place independently requests data required for the operation of the transmission and reception elements (5) from the transmission buffer (20) and uses that data within the context of the real-time bus system (BUS-S) and, secondly, writes measurement data independently into the reception buffer (21).

**15.** The ultrasound method according to any one of claims 10 to 14, **characterised in that** when the ultrasound probe (2) is switched on and when the connection to the data processing unit (3) is broken off, the ultrasound probe (2) independently assumes a safe default state.

## Revendications

**1.** Système à ultrasons, pourvu d'une sonde ultrasonique (2), laquelle comprend une pluralité d'éléments émetteurs et récepteurs (5) et pourvu d'une unité de traitement de données (3) physiquement séparée de la sonde ultrasonique (2) et connectée avec celle-ci par technique de données (14), la sonde ultrasonique (2) comportant un nombre de préamplificateurs (8) analogiques, plusieurs convertisseurs analogique-numérique (9), ainsi qu'au moins un composant de raccordement (11, 12, 13) numérique et l'unité de traitement de données (3) comportant deux mémoires annulaires (20, 21), à savoir une mémoire réceptrice (21) et une mémoire émettrice (20), ainsi qu'une unité de calcul (15, 16, 17, 19, 22) conçue pour la génération d'images ultrasoniques, et pour le couplage par transfert de données de la sonde ultrasonique (2) avec l'unité de calcul (15, 16, 17, 19, 22) étant prévu un système de bus (Bus-S) en temps réel, comprenant la dénommée connexion de données (14), dans lequel sont également intégrées les mémoires annulaires (20, 21) et qui est relié d'une part à un système de sonde (Son-S) conduisant des données à l'intérieur de la sonde ultrasonique (2) et d'autre part à un système d'exploitation (BS), destiné à faire fonctionner l'unité de calcul (15, 16, 17, 19, 22), et l'incertitude de l'horloge (gigue) du système de sonde (Son-S) étant plus faible d'au moins trois ordres de grandeur et le temps de réaction du système d'exploitation (BS) étant plus long d'au moins deux ordres de grandeur que l'incertitude de l'horloge (gigue) du système de bus (Bus-S) en temps réel, et le système de bus (Bus-S) en temps réel en interaction avec le système de sonde (Son-S) et le système d'exploitation (BS) étant conçu pour transmettre des données d'émission et de réception simultanément par streaming de manière ininterrompue, avec un débit constant dans la moyenne, de manière cadencée en fonctionnement sans statut, ne différenciant pas le fonctionnement en émission et en réception de la sonde ultrasonique (2).

**2.** Système à ultrasons selon la revendication 1, **caractérisé en ce que** par la dénommée connexion de données (14), une connexion est établie entre la sonde ultrasonique (2) et un root complex (15) intégré dans l'unité de traitement de données (3), lequel est prévu entre autres pour transmettre des paquets de données (MSI) de messages, concernant des données d'émission ains que de réception, par les paquets de données (MSI) de messages étant réalisés des sémaphores de comptage, pour le blocage temporel et la libération de la transmissions de données en streaming dans la mémoire émettrice (20) ainsi qu'à partir de la mémoire réceptrice (21).

**3.** Système à ultrasons selon la revendication 2, **ca-**

**ractérisé en ce qu'**en amont de chaque élément émetteur et récepteur (5) est monté un duplexeur émetteur-récepteur (6), qui est conçu pour soumettre en cadence l'élément émetteur et récepteur (5) pour chaque fois précisément une cadence à l'un parmi n codes, n correspondant au moins à quatre et (n-1) codes d'émission, auxquels sont associés des valeurs de tension et précisément un code de réception étant prévus.

4.  Système à ultrasons selon la revendication 2 ou 3, **caractérisé en ce que** la capacité de transmission de données tenue à disposition pour la transmission de paquets de données (MSI) de messages ne s'élève pas à plus de chaque fois 1 % des capacités de transmission de données, lesquelles sont mises à disposition pour la transmission, d'une part de données d'adressage et d'autre part de données de mesure via la connexion de données (14).

5.  Système à ultrasons selon l'une quelconque des revendications 1 à 4, **caractérisé en ce qu'**entre la gigue temporelle (TSon) du système de sonde (Son-S), la gigue (TBus) du système de bus (Bus-S) en temps réel, et le temps de réaction (TBS) du système d'exploitation (BS), il existe la relation suivante :

$$10^1 \leq \frac{TBus^2}{TSoN*TBS} \leq 10^4$$

6.  Système à ultrasons selon l'une quelconque des revendications 1 à 5, **caractérisé en ce que** la mémoire réceptrice (21) est conçue pour mémoriser pendant au moins 20 ms des blocs de données de réception, lesquels sont associés à des jeux de données d'émission.

7.  Système à ultrasons selon la revendication 6, **caractérisé en ce que** le flux de données d'émission est prévu pour l'adressage ininterrompu continu des éléments émetteurs et récepteurs (5) et **en ce que** les données de réception connexes sauvegardées dans les mémoires émettrice et réceptrice (20, 21) sont mémorisées de manière séquentielle, sans lacune temporelle et sans signal de temps connexe au cas par cas.

8.  Système à ultrasons selon l'une quelconque des revendications 1 à 7, **caractérisé en ce que** la connexion de données (14) entre la sonde ultrasonique (2) et les mémoires annulaires (20, 21) est conçue sous la forme d'une connexion PCIe.

9.  Système à ultrasons selon la revendication 8, **caractérisé en ce que** la connexion de données comprend un câble (14) connectant la sonde ultrasonique (2) avec l'unité de traitement de données (3).

10. Procédé d'imagerie par ultrasons, lors duquel un objet qui doit être examiné est exposé à des impulsions ultrasoniques et des signaux d'écho sont enregistrés, présentant les caractéristiques suivantes :

    - la mise à disposition d'une sonde ultrasonique (2), laquelle comprend une pluralité d'éléments émetteurs et récepteurs (5), ainsi que d'une unité de traitement de données (3) couplée avec celle-ci par technique de données, laquelle comprend un processeur principal (17), ainsi qu'un processeur (22), notamment un processeur graphique, pourvu d'une pluralité de calculateurs (23),

    - la génération par l'unité de traitement de données (3) de données d'adressage prévues pour le fonctionnement des éléments émetteurs et récepteurs (5), les données d'adressage étant créées sous un système d'exploitation (BS) standard ne satisfaisant pas à des exigences sévères de temps réel et étant écrites de manière asynchrone (en ce qui concerne la création) dans une mémoire émettrice (20) conçue sous la forme de mémoire annulaire, qui est attribuable à un système de bus (BUS-S) en temps réel,

    - la transmission asynchrone (en ce qui concerne la génération et la mémorisation des données d'adressage) de données d'adressage à partir de la mémoire émettrice (20) à la sonde ultrasonique (2), la sonde ultrasonique (2) étant utilisée en tant que composant du système de bus (BUS-S) en temps réel et les données d'adressage étant transmises avec un premier débit de données constant dans la moyenne, sans pause par streaming et l'incertitude de l'horloge (gigue) d'un système de sonde (Son-S) comprenant des composants à l'intérieur de la sonde ultrasonique étant plus faible d'au moins trois ordres de grandeur et le temps de réaction du système d'exploitation (BS) standard étant plus long d'au moins deux ordres de grandeur que l'incertitude de l'horloge (gigue) du système de bus (Bus-S) en temps réel,

    - les impulsions ultrasoniques sont créées selon les critères des données d'adressage à l'aide d'étages de sortie (7) d'émission placés dans la sonde ultrasonique (2), les éléments émetteurs et récepteurs (5) actionnés par les étages de sortie (7) d'émission étant réalisés notamment sous la forme d'élément piézoélectriques,

    - les signaux d'écho connexe sont amplifiés de manière analogique à l'intérieur de la sonde ultrasonique (2) et numérisés de manière synchrone, sans interruption et à une fréquence d'horloge constante, et écrits de manière asynchrone par streaming en temps réel sans lacune, sans traitement supplémentaire, via une connexion de données (14) dans une mémoire réceptrice (21), spatialement séparée de la sonde ultrasonique (2), conçue en tant que mémoire annulaire, attribuable au système de bus (BUS-S) en temps réel, la transmission de données vers la mémoire réceptrice (21) s'effectuant sans interruption, avec un deuxième débit de données sensiblement constant, lequel diverge de pas plus d'un coefficient cinq du premier débit de données concernant la transmission de données de l'unité de traitement de données (3) vers la sonde ultrasonique (2),

- une sélection de données qui sont entrées dans la mémoire réceptrice (21) est commandée par le processeur principal (17), distribuée en tant que blocs de données de mesure sur plusieurs calculateurs (23), les blocs individuels de données de mesure étant affectés chacun à un groupe d'éléments de réception (5),

- plusieurs jeux de données de mesure sont traités de manière parallèle et asynchrone au processus d'écriture, c'est-à-dire pour l'écriture dans la mémoire réceptrice (21) du système de bus (BUS-S) en temps réel par des calculateurs (23) qui sont commandés chacun par le processeur principal (17) fonctionnant sous le système d'exploitation (BS) standard, le traitement comprenant respectivement la formation du faisceau de réception et la reconstruction d'image et à l'aide d'un calculateur (23), une image partielle étant reconstruite,

- à partir des images partielles, une image globale est générée en tant qu'image ultrasonore par l'unité de traitement de données (3).

11. Procédé par ultrasons selon la revendication 10, **caractérisé en ce qu'**additionnellement à des données d'adressage et à des données de mesure fournies par la sonde ultrasonique (2), à l'intérieur de l'unité de traitement de données (3) sont transmis des paquets de données (MSI) de messages, lesquels réalisent des sémaphores de comptage pour le blocage et la libération de la transmission sérielle de données par streaming dans la mémoire émettrice (20) ainsi qu'à partir de la mémoire réceptrice (21), le volume de données des paquets de données (MSI) de messages étant inférieur d'au moins deux ordres de grandeur au volume des données conduites via les mémoires annulaires (20, 21).

12. Procédé par ultrasons selon la revendication 10 ou 11, **caractérisé en ce que** les signaux d'écho de chacun des canaux récepteurs à l'intérieur de la sonde ultrasonique (2) sont amplifiés de manière analogique avec une amplification (gain) constante par un préamplificateur (8).

13. Procédé par ultrasons selon l'une quelconque des revendications 10 à 12, **caractérisé en ce que** dans les mémoires émettrice et réceptrice (20, 21), des données sont écrites sans information temporelle explicite, lors de la lecture des données, l'information temporelle étant prélevée implicitement à partir de la grandeur des blocs de données, ainsi que de l'ordre chronologique de mémorisation, c'est-à-dire de l'adresse de mémorisation.

14. Procédé par ultrasons selon l'une quelconque des revendications 10 à 13, **caractérisé en ce que** premièrement, la sonde ultrasonique (2) demande automatiquement à partir de la mémoire émettrice (20) des données pour le fonctionnement des éléments émetteurs et récepteurs (5) et les exploite dans le cadre du système de bus (BUS-S) en temps réel et deuxièmement, écrit indépendamment des données de mesure dans la mémoire réceptrice (21).

15. Procédé par ultrasons selon l'une quelconque des revendications 10 à 14, caractérisé en ce lors de l'initiation ainsi que de la rupture de la connexion avec l'unité de traitement de données (3), la sonde ultrasonique (2) adopte automatiquement un état de défaut sûr.

# Fig. 1

# Fig. 2

BS

S1

S2

S3

20

S4

Son-S

S5

S6

S7

S8

21

S9

S10

BS

S11

S12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130079639 A1
- EP 2614775 A1
- US 20080110266 A1
- US 20080294046 A1
- US 20140058266 A1
- EP 2627257 B1
- EP 3117774 B1
- US 20080151765 A1
- DE 102018203444 A1
- EP 0762142 B1